# EUROPÄISCHE PATENTSCHRIFT

(11) **EP 4 001 256 B1**
(45) Veröffentlichungstag und Bekanntmachung des Hinweises auf die Patenterteilung: **22.07.2026**
(21) Anmeldenummer: 20209477.7
(22) Anmeldetag: 24.11.2020
(51) Int. Cl.: C07C 67/38, C07C 69/24

(54) **VERFAHREN ZUR ALKOXYCARBONYLIERUNG VON ETHYLENISCH UNGESÄTTIGTER VERBINDUNGEN UNTER EINSATZ VON BENZOLBASIERTE DIPHOSPHINLIGANDEN UND ALUMINIUMTRIFLAT**
METHOD FOR ALKOXYCARBONYLATION OF ETHYLENICALLY UNSATURATED COMPOUNDS USING BENZENE-BASED DIPHOSPHINE LIGANDS AND ALUMINUM TRIFLATE
PROCÉDÉ D'ALCOXYCARBONYLATION DE COMPOSÉS ÉTHYLÉNIQUEMENT INSATURÉS À L'AIDE DES LIGANDS DE DIPHOSPHINE À BASE DE BENZÈNE ET DU TRIFLATE D'ALUMINIUM

(43) Veröffentlichungstag der Anmeldung: 25.05.2022
(73) Patentinhaber: Evonik Oxeno GmbH & Co. KG, 45772 Marl (DE)
(72) Erfinder: KUCMIERCZYK, Peter, 44628 Herne (DE); DÜHREN, Ricarda, 18069 Rostock (DE); FRIDAG, Dirk, 45721 Haltern am See (DE); KNOSSALLA, Johannes, 46514 Schermbeck (DE); SALE, Anna Chiara, 45657 Recklinghausen (DE); MARKOVIC, Ana, 45721 Haltern am See (DE); BRÄCHER, Alexander, 45721 Haltern am See (DE); BELLER, Matthias, 18211 Ostseebad (DE); FRANKE, Robert, 45772 Marl (DE)
(74) Vertreter: Evonik Patent Association

(56) Entgegenhaltungen:
- EP-A1- 3 750 620
- DONG KAIWU ET AL: "Efficient Palladium-Catalyzed Alkoxycarbonylation of Bulk Industrial Olefins Using Ferrocenyl Phosphine Ligands", vol. 56, no. 19, 2 May 2017 (2017-05-02), pages 5267 - 5271, XP055801046, ISSN: 1433-7851, Retrieved from the Internet <URL:https://api.wiley.com/onlinelibrary/tdm/v1/articles/10.1002%2Fanie.201700317> DOI: 10.1002/anie.201700317
- WILLIAMS D BRADLEY G ET AL: "Aluminum Triflate as a Highly Active and Efficient Nonprotic Cocatalyst in the Palladium-Catalyzed Methoxycarbonylation Reaction", vol. 47, no. 3, 1 January 2008 (2008-01-01), pages 560 - 563, XP002674851, ISSN: 1433-7851, Retrieved from the Internet <URL:http://onlinelibrary.wiley.com/doi/10.1002/anie.200702889/abstract?systemMessage=Wiley+Online+Library+will+be+disrupted+on+26+May+from+10%3A00-12%3A00+BST+%2805%3A00-07%3A00+EDT%29+for+essential+maintenance> DOI: 10.1002/ANIE.200702889

## Beschreibung

Die Erfindung betrifft ein Verfahren zur Alkoxycarbonylierung von ethylenisch ungesättigter Verbindungen unter Einsatz von benzol basierte Diphosphinliganden und Aluminiumtriflat.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

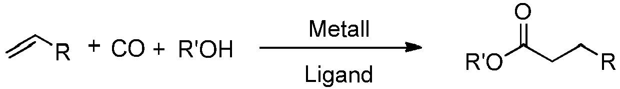

In Dong Kaiwu et al.: "Efficient Palladium-Catalyzed Alkoxycarbonylation of Bulk Industrial Olefins Using Ferrocenyl Phosphine Ligands" Angewandte Chemie International Edition 2017, Vol: 56, Nr: 19, Seiten: 5267 - 5271, wird ein Verfahren zur Alkoxycarbonylierung von Olefinen beschrieben. Das Verfahren wird durch einen Palladium-Ligand-Komplex katalysiert.

EP 3 121 184 A2 beschreibt ein Verfahren zur Alkoxycarbonylierung von Olefinen unter Einsatz von benzolbasierten Diphosphinverbindungen. Der Reaktion wurde hierbei jeweils eine Brønsted-Säure zugesetzt: para-Toluolsulfonsäure (PTSA), Trifluormethansulfonsäure oder Schwefelsäure.

Schwefelsäure verursacht allerdings auf metallischen Oberflächen eine starke Korrosion. Ein weiterer Nachteil, welcher der Einsatz von Schwefelsäure mit sich bringt, ist, dass diese aufwendig vorbehandelt / entgast werden muss.

Katalysatorsystem sind häufig sensitiv gegenüber Sauerstoff. Der Kontakt selbst mit Spuren an Sauerstoff führt zur Oxidation des Liganden was schlussendlich zu einer Verringerung der Aktivität des gesamten Katalysatorkomplexes führt. Spuren an Sauerstoff können beispielsweise durch das kontinuierliche Einbringen von Komponenten miteingetragen werden.

Die technische Aufgabe der Erfindung ist die Bereitstellung eines neuen Verfahrens, welches die in Zusammenhang mit dem Einsatz, der aus dem Stand der Technik genannten Brønsted-Säure, nicht aufweist. Des Weiteren soll das Verfahren eine gute Ausbeute liefern.

Die Aufgabe wird gelöst durch ein Verfahren nach Anspruch 1.

Verfahren umfassend die Verfahrensschritte:
a) Vorlegen einer ethylenisch ungesättigten Verbindung;
b) Zugabe eines Liganden gemäß Formel (I): 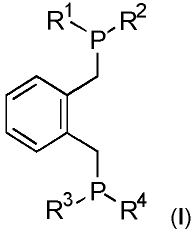 wobei
   R¹ und R³ jeweils für einen -(C₃-C₂₀)-Heteroarylrest stehen,
   R² und R⁴ jeweils für -(C₁-C₁₂)-Alkyl stehen,
   und einer Verbindung, welche Pd umfasst;
c) Vorbehandlung von Aluminiumtriflat durch anlegen von Vakuum;
d) Zugabe des vorbehandelten Aluminiumtriflats aus c),
   wobei das Verhältnis Aluminiumtriflat : Ligand im Bereich von 2 mol : 1 mol bis 25 mol : 1 mol liegt;
e) Zugabe eines Alkohols;
f) Zuführen von CO;
g) Erwärmen des Reaktionsgemisches aus a) bis f), wobei die ethylenisch ungesättigte Verbindung zu einem Ester umgesetzt wird.

Hierbei kann die Zugabe der Stoffe in beliebiger Reihenfolge erfolgen. Üblicherweise erfolgt die Zugabe von CO jedoch, nachdem die Reaktionspartner in den Schritten a) bis e) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Unter "Vakuum" wird in Zusammenhang mit dieser Erfindung ein Druck von 100 mbar oder weniger verstanden.

Die Vorbehandlung des Aluminiumtriflats kann beispielsweise in einem Vorlagebehälter erfolgen.

Das Anlegen von Vakuum kann hierbei mehrmals wiederholt werden.

In einer Variante des Verfahrens wird der Verfahrensschritt c) mindestens zweimal durchgeführt, und das angelegte Vakuum durch Fluten mit Inertgas aufgehoben. Als Inertgas kann beispielsweise N₂, He oder Ar verwendet werden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In dem Fall, dass der Katalysator *in situ* gebildet wird, kann der Ligand im Überschuss zugegeben werden, so dass im Reaktionsgemisch auch ungebundener Ligand vorliegt.

In einer Variante des Verfahrens sind R¹, R³ R¹, R³ jeweils ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

In einer Variante des Verfahrens stehen R² und R⁴ für *^{ter}*Bu.

In einer Variante des Verfahrens weist der Ligand in Verfahrensschritt b) die Formel (1) auf:

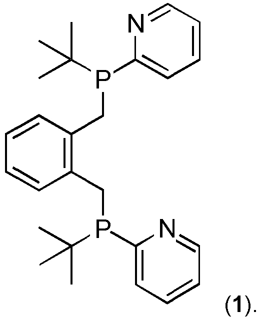

In einer Variante des Verfahrens umfasst das Verfahren den zusätzlichen Verfahrensschritt c'):
c') Lösen des vorbehandelten Aluminiumtriflat aus c) in einem Lösungsmittel.

Durch das Herstellen einer Lösung aus dem vorbehandelten Aluminiumtriflat ist es möglich, dem Verfahren kontinuierlich Aluminiumtriflat zuzudosieren, ohne dass hierbei signifikante Mengen an Sauerstoff eingeschleppt werden.

In einer Variante des Verfahrens handelt es sich bei dem Lösungsmittel im Verfahrensschritt c') um einen Alkohol.

In einer Variante des Verfahrens wird als Lösungsmittel im Verfahrensschritt c') der gleiche Alkohol wie in Verfahrensschritt e) verwendet.

In einer Variante des Verfahrens ist die Verbindung in Verfahrensschritt b), welche Pd umfasst, ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

Vorzugsweise handelt es sich bei der Verbindung, welche Pd umfasst, um Pd(dba)₂, Pd(acac)₂ oder Pd(OAc)₂. Besonders geeignet ist Pd(acac)₂.

Das Massenverhältnis von Pd zu der in Schritt a) vorgelegten ethylenisch ungesättigten Verbindung liegt vorzugsweise in Bereich von 0,001 bis 0,5 Gew.-%, bevorzugt von 0,01 bis 0,1 Gew.-%, besonders bevorzugt von 0,01 bis 0,05 Gew.-%.

Das molare Verhältnis des Liganden zu Pd liegt vorzugsweise im Bereich von 0,1:1 bis 400:1, bevorzugt von 0,5:1 bis 400:1, besonders bevorzugt von 1:1 bis 100:1, am meisten bevorzugt von 2:1 bis 50:1.

In einer Variante des Verfahrens ist der Alkohol in Verfahrensschritt e) ausgewählt aus Methanol, Ethanol, 1-Propanol, 1-Butanol, 1-Pentanol, 1-Hexanol, 2-Propanol, tert-Butanol, 3-Pentanol, Cyclohexanol, Phenol, oder Mischungen davon.

In einer Variante des Verfahrens ist der Alkohol in Verfahrensschritt e) Methanol.

In einer Variante des Verfahrens wird der Alkohol im Verfahrensschritt e) im Überschuss eingesetzt.

In einer Variante des Verfahrens wird der Alkohol im Verfahrensschritt e) gleichzeitig als Lösungsmittel eingesetzt.

Das molare Verhältnis von der in Verfahrensschritt a) vorgelegten ethylenisch ungesättigten Verbindung zu dem in Verfahrensschritt e) zugegebenen Alkohol liegt vorzugsweise im Bereich von 1:1 bis 1:20, bevorzugt von 1:2 bis 1:10, besonders bevorzugt von 1:3 bis 1:6.

In einer Variante des Verfahrens liegt das Verhältnis Aluminiumtriflat : Ligand in Verfahrensschritt d) im Bereich von 2,5 mol : 1 mol bis 15 mol : 1 mol.

CO wird in Verfahrensschritt f) vorzugsweise bei einem CO-Partialdruck im Bereich von 0,1 bis 10 MPa (1 bis 100 bar), bevorzugt von 1 bis 5 MPa (10 bis 50 bar), besonders bevorzugt von 1 bis 2 MPa (10 bis 20 bar) zugeführt.

Die Reaktionsmischung wird in Verfahrensschritt g) des erfindungsgemäßen Verfahrens vorzugsweise auf eine Temperatur im Bereich von 30 °C bis 150 °C, bevorzugt von 40 °C bis 140 °C, besonders bevorzugt von 50 °C bis 120 °C erwärmt, um die ethylenisch ungesättigte Verbindung zu einem Ester umzusetzen.

Im Folgenden soll die Erfindung anhand von Ausführungsbeispielen näher erläutert werden.

### Allgemeine Arbeitsvorschriften

Wenn nicht anders angegeben wird unter Argon Atmosphäre gearbeitet. Reaktionsgefäße sind zuvor unter Einwirkung von Temperatur (80 °C) und Ölpumpenvakuum getrocknet worden.

Flüssige Substanzen (z.B. Schwefelsäure (H₂SO₄)) werden für mind. 15 Minuten durch einperlen von Argon entgast.

Das eingesetzte Aluminiumtrifalt (Al(OTf)₃) und auch andere feste Säuren wurden wie folgt vorbehandelt: Im Falle einer festen Säuren wird diese zunächst eingewogen und das Gefäß luftdicht mittels eines gebördelten Septums verschlossen. Mittels durchstochener Kanüle, welche an eine Argon-/Vakuum-Verteilerstation (Schlenkline) angeschlossen ist, wird zunächst durch dreimaliges abwechselndes anlegen von Vakuum (50 mbar) und fluten mit Argon diese Säure sauerstofffrei vorbereitet. Zusätzlich wird in den folgenden Schritten Argonatmosphäre garantiert und besteht gleichzeitig die Möglichkeit des Druckausgleichs (Zugabe Lösungen).

Als Ligand (**1**) wird 1,2-Bis((tert-butyl(pyridin-2-yl)phosphanyl)methyl)benzol eingesetzt. Als Präkursor wird Palladium(II)bis(acetylacetonate) (Pd(acac)₂) eingesetzt. Di-iso-buten stellt ein Gemisch bestehend aus den beiden C8-Isomeren 2,4,4-Trimethylpent-1-en und 2,4,4-Trimethylpent-2-en in den Verhältnissen von ungefähr 80:20 dar. 0,5 mL dieser Proben werden mit Isooctan als interner Standard versetzt und der Umsatz und die Ausbeute mittels GC und GCMS Analytik bestimmt.

### Analytik

GC Analytik Di-iso-Buten und 1-Octen: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1.

### Experimente

### Variation der Lewissäuren (1-Octene)

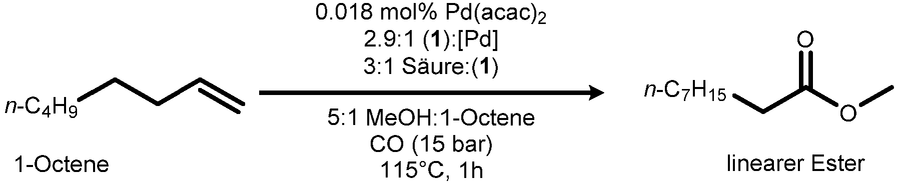

### Katalysatorlösung:

In einem 10 mL Schlenkgefäß wird Pd(acac)₂ (8,53 mg) und (**1**) (35,42 mg) eingewogen und in Methanol gelöst (7 mL).

### Schwefelsäurelösung:

In einem 15 mL Schlenkgefäß wird H₂SO₄ (0,184 g) eingewogen und in Methanol gelöst (10 mL).

Die Reaktion wird in 10mL Glasgefäßen mit Magnetrührfischen durchgeführt. Im Falle einer festen Säure wird diese zunächst eingewogen (späteres Verhältnis von Säure : (**1**) soll 3 mol :1 mol betragen) und wie zuvor beschrieben vorbehandelt. Im Fall von Salicyclsäure werden beispielsweise 10,76 mg, 0,156 mol% eingewogen. Die benötigte Menge an Katalysatorlösung (0,75 mL) wird mittels µL-Spritze zugegeben, sodass sich eine Einwaage von Pd(acac)₂ (0,914 mg, 0,018 mol%) und (**1**) (3,795 mg, 0,052 mol%) ergibt. Für Untersuchungen mit flüssigen Säuren wird die benötigte Menge an Säurelösung hinzugeben. Beispielsweise wird für ein H₂SO₄-Verhältnisses von 3:1 0,14 mL (0,156 mol%) mittels µL-Spritze hinzugegeben. Abschließend wird Methanol mittels µL-Spritze hinzugegeben damit ein Gesamtvolumen von 3,38 mL vorliegen und sich ein molares MeOH zu Substrat Verhältnis von 5:1 ergibt. Im zuvor genannten Beispiel werden demnach 2,3 mL zugegeben. Fünf der vorbereiteten Glasgefäße werden in einem 300 mL Autoklaven aufgehangen. Gleichzeitig wird in jedes Gefäß eine separate Leitung geführt, die eine definierte zu Dosierung des Substrates bei Reaktionstemperatur ermöglicht. Der Autoklav wird verschlossen und dreimal mit CO gespült und dann mit 15 bar CO aufgepresst. Die Reaktionslösungen werden dann auf die benötigte Temperatur von 115 °C aufgeheizt. Nach 20 Minuten bei konstanter Temperatur wird das Substrat mittels HPLC-Pumpe in die Reaktionsgefäße überführt (2,6 mL, 16,7 mmol). Nach 1 h wird über die Substratleitung jeweils eine Probe gezogen. 0,5mL dieser Probe werden mit Isooctan als Standard versetzt und Ausbeute und n:iso Verhältnisse mittels GC GCMS-Analyse bestimmt.

| Säure | Ausbeute (Ester-Gemisch) [%] | n:iso [%] |
|---|---|---|
| H₂SO₄ | 78 | 68 |
| Al(OTf)₃* | 92 | 67 |
| Salicylsäure | 1 | 68 |
| B(OH)₃ | 4 | 68 |
| BSA (0,156 mol%) | 4 | 70 |
| [B(OH)₃]/[Salicylic acid] 1:2 | | |
| BSA (0,520 mol%) | 6 | 70 |
| [B(OH)₃]/[Salicylic acid] 1:2 | | |
| BSA (1,040 mol%) | 5 | 70 |
| [B(OH)₃]/[Salicylic acid] 1:2 | | |
| Borsäure-trimethylester | 2 | 68 |
| Borsäure-methylester | 3 | 67 |
| 2-Thienylboransäure | 2 | 68 |
| Phenylboransäure | 2 | 68 |
| Tris(pentafluorophenyl)borane | 70 | 67 |
| Ce(SO₄)₂ | 2 | 67 |

| | | |
|---|---|---|
| * erfindungsgemäße Ausführungsbeispiele | | |

### Aluminium oder Triflatverbindungen (Di-iso-buten)

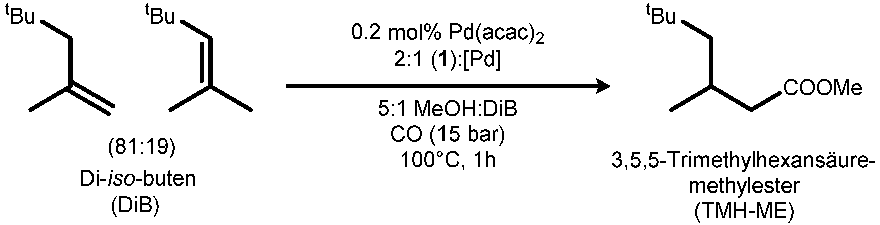

### Katalysatorlösung:

In einem 10 mL Schlenkgefäß wird Pd(acac)₂ (83,3 mg) und (**1**) (238,1 mg) eingewogen und in Methanol gelöst (7 mL).

### Schwefelsäurelösung:

In einem 15 mL Schlenkgefäß wird H₂SO₄ (0,386 g) eingewogen und in Methanol gelöst (5 mL).

Die Reaktion wird in 10mL Glasgefäßen mit Magnetrührfischen durchgeführt. Im Falle einer festen Säure wird diese zunächst eingewogen (4 mol%, molares Verhältnis Säure : (**1**) = 10:1) und wie zuvor beschrieben vorbehandelt. Die benötigte Menge an Katalysatorlösung (1 mL) wird mittels µL-Spritze zugegeben, sodass sich eine Einwaage von Pd(acac)₂ (11,9 mg, 0,2 mol%) und (**1**) (34,01 mg, 0,4 mol%) ergibt. Für Untersuchungen mit der flüssigen Säure H₂SO₄ wird die benötigte Menge an Säurelösung 1 mL (4 mol%) mittels µL-Spritze hinzugegeben. Abschließend wird Methanol mittels µL-Spritze hinzugegeben damit ein Gesamtvolumen von 3,94 mL vorliegt und sich ein molares MeOH zu Substrat Verhältnis von 5:1 ergibt. Fünf der vorbereiteten Glasgefäße werden in einem 300 mL Autoklaven aufgehangen. Gleichzeitig wird in jedes Gefäß eine separate Leitung geführt, die eine definierte Zudosierung des Substrates (3 mL, 19,4 mmol) bei Reaktionstemperatur ermöglicht. Der Autoklav wird verschlossen und dreimal mit CO gespült und dann mit 15 bar CO aufgepresst. Die Reaktionslösungen werden dann auf die benötigte Temperatur von 115 °C aufgeheizt. Nach 20 Minuten bei konstanter Temperatur wird das Substrat mittels HPLC-Pumpe in die Reaktionsgefäße überführt (3 mL, 19,4 mmol). Nach 1 h wird über die Substratleitung jeweils eine Probe gezogen. 0,5 mL dieser Probe werden mit Isooctan als Standard versetzt und Ausbeute und n:iso Verhältnisse mittels GC GCMS-Analyse bestimmt.

| Säure | Ausbeute (TMH-ME) [%] |
|---|---|
| H₂SO₄ | 87 |
| Al(OTf)₃* | 94 |
| Cu(OTf)₃ | <1 |
| Fe(OTf)₃ | <1 |
| Mg(OTf)₃ | <1 |
| Na(OTf)₃ | <1 |
| Zn(OTf)₃ | <1 |
| Al(H₂PO₄)₃ | 3 |
| Al₂(SO₄)₃ | 8 |
| Al(acac)₃ | <1 |

| | |
|---|---|
| * erfindungsgemäße Ausführungsbeispiele | |

### Variation des Liganden (1-Octene)

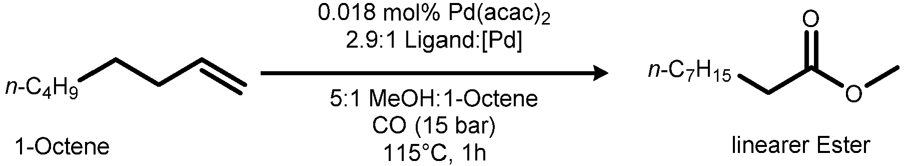

### Katalysatorlösung:

In einem 10 mL Schlenkgefäß wird Pd(acac)₂ (0,004 mol/L) und Ligand (0,0116 mol/L) eingewogen für 7 mL Methanol-Lösung.

Die Reaktion wird in 10mL Glasgefäßen mit Magnetrührfischen durchgeführt. Im Falle einer festen Säure wird diese zunächst eingewogen und wie zuvor beschrieben vorbehandelt. Im Beispiel des benötigten Verhältnisses von 1mol:1mol wird 4,13 mg Al(OTf)₃ eingewogen. Die benötigte Menge an Katalysatorlösung (0,75 mL) wird mittels µL-Spritze zugegeben, sodass sich eine Einwaage von Pd(acac)₂ (0,914 mg, 0,018 mol%) und (**1**) (3,795 mg, 0,052 mol%) ergibt. Abschließend wird Methanol mittels µL-Spritze hinzugegeben damit ein Gesamtvolumen von 3,38 mL vorliegen und sich ein molares MeOH zu Substrat Verhältnis von 5:1 ergibt. Fünf der vorbereiteten Glasgefäße werden in einem 300 mL Autoklaven aufgehangen. Gleichzeitig wird in jedes Gefäß eine separate Leitung geführt, die eine definierte zu Dosierung des Substrates bei Reaktionstemperatur ermöglicht. Der Autoklav wird verschlossen und dreimal mit CO gespült und dann mit 15 bar CO aufgepresst. Die Reaktionslösungen werden dann auf die benötigte Temperatur von 115 °C aufgeheizt. Nach 20 Minuten bei konstanter Temperatur wird das Substrat mittels HPLC-Pumpe in die Reaktionsgefäße überführt (2,6 mL, 16,7 mmol). Nach 1 h wird über die Substratleitung jeweils eine Probe gezogen. 0,5mL dieser Probe werden mit Isooctan als Standard versetzt und Ausbeute und n:iso Verhältnisse mittels GC GCMS-Analyse bestimmt.

| Ligand | Säure: Ligand [mol]:[mol] | Ausbeute (Ester-Gemisch) [%] | n:iso [%] |
|---|---|---|---|
| 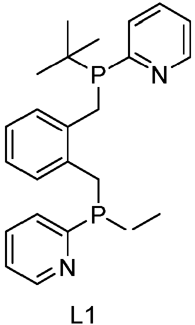 | 0,5 : 1 | 70 | 68 |
| | 1 : 1 | 82 | 68 |
| | 3 : 1* | 92 | 68 |
| | 5 : 1* | 89 | 68 |
| | 7 : 1 * | 88 | 68 |
| | 10 : 1* | 88 | 68 |
| 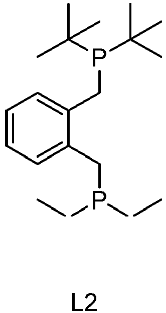 | 3 : 1 | 19 | 92 |
| | 5 : 1 | 17 | 92 |
| | 10 : 1 | 15 | 92 |
| | 15 : 1 | 17 | 92 |
| 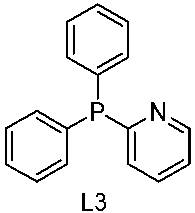 | 1 : 1 | 9 | 72 |
| | 3 : 1 | 10 | 72 |
| | 5 : 1 | 10 | 72 |
| | 7,5 : 1 | 11 | 72 |
| 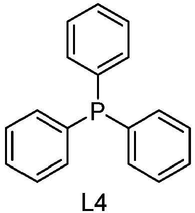 | 1 : 1 | 54 | 69 |
| | 3 : 1 | 65 | 67 |
| | 5 : 1 | 76 | 68 |
| | 7,5 : 1 | 76 | 68 |

| | | | |
|---|---|---|---|
| * erfindungsgemäße Ausführungsbeispiele | | | |

### Variation der Säureäquivalente (1-Octene)

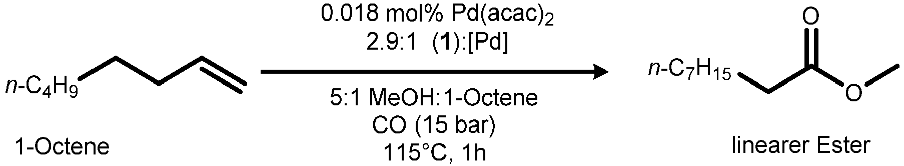

### Katalysatorlösung:

In einem 10 mL Schlenkgefäß wird Pd(acac)₂ (8,53 mg) und (1) (35,42 mg) eingewogen und in Methanol gelöst (7 mL).

### Schwefelsäurelösung:

In einem 15 mL Schlenkgefäß wird H₂SO₄ (0,184 g) eingewogen und in Methanol gelöst (10 mL).

Die Reaktion wird in 10mL Glasgefäßen mit Magnetrührfischen durchgeführt. Im Falle einer festen Säure wird diese zunächst eingewogen und wie zuvor beschrieben vorbehandelt. Die benötigte Menge an Katalysatorlösung (0,75 mL) wird mittels µL-Spritze zugegeben, sodass sich eine Einwaage von Pd(acac)₂ (0,914 mg, 0,018 mol%) und (**1**) (3,795 mg, 0,052 mol%) ergibt. Für Untersuchungen mit flüssigen Säuren wird die benötigte Menge an Säurelösung hinzugeben. Beispielsweise wird für ein H₂SO₄-Verhältnisses von 4:1 0,19 mL (0,21 mol%) mittels µL-Spritze hinzugegeben. Abschließend wird Methanol mittels µL-Spritze hinzugegeben damit ein Gesamtvolumen von 3,38 mL vorliegen und sich ein molares MeOH zu Substrat Verhältnis von 5:1 ergibt. Im zuvor genannten Beispiel werden demnach 2,44 mL zugegeben. Fünf der vorbereiteten Glasgefäße werden in einem 300 mL Autoklaven aufgehangen. Gleichzeitig wird in jedes Gefäß eine separate Leitung geführt, die eine definierte zu Dosierung des Substrates bei Reaktionstemperatur ermöglicht. Der Autoklav wird verschlossen und dreimal mit CO gespült und dann mit 15 bar CO aufgepresst. Die Reaktionslösungen werden dann auf die benötigte Temperatur von 115 °C aufgeheizt. Nach 20 Minuten bei konstanter Temperatur wird das Substrat mittels HPLC-Pumpe in die Reaktionsgefäße überführt (2,6 mL, 16,7 mmol). Nach 1 h wird über die Substratleitung jeweils eine Probe gezogen. 0,5 mL dieser Probe werden mit Isooctan als Standard versetzt und Ausbeute und n:iso Verhältnisse mittels GC GCMS-Analyse bestimmt.

| Säure | Säure:(**1**) [mol:mol] | Ausbeute (Ester-Gemisch) [(%] |
|---|---|---|
| H₂SO₄ | 1 : 1 | 44 |
| | 2 : 1 | 73 |
| | 3 : 1 | 78 |
| | 4 : 1 | 73 |
| | 5 : 1 | 65 |
| | 7 : 1 | 48 |
| | 10 : 1 | 34 |
| Al(OTf)₃ | 1 : 1 | 82 |
| | 3 : 1* | 92 |
| | 5 : 1* | 89 |
| | 7 : 1 * | 88 |
| | 10 : 1* | 88 |

| | | |
|---|---|---|
| * erfindungsgemäße Ausführungsbeispiele | | |

### Variation der Säureäquivalente (Di-iso-buten)

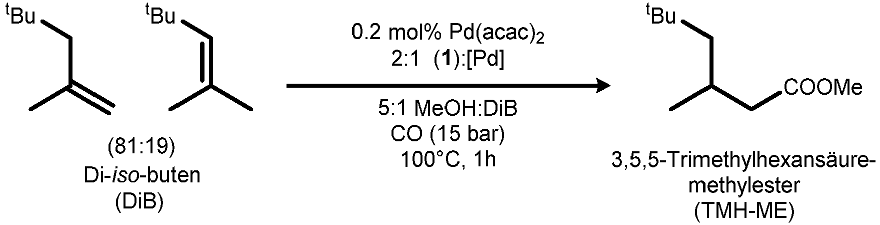

### Katalysatorlösung:

In einem 10 mL Schlenkgefäß wird Pd(acac)₂ (83,3 mg) und (**1**) (238,1 mg) eingewogen und in Methanol gelöst (7 mL).

### Schwefelsäurelösung:

In einem 15 mL Schlenkgefäß wird H₂SO₄ (0,386 g) eingewogen und in Methanol gelöst (5 mL).

Die Reaktion wird in 10mL Glasgefäßen mit Magnetrührfischen durchgeführt. Im Falle einer festen Säure wird diese zunächst eingewogen und wie zuvor beschrieben vorbehandelt. Die benötigte Menge an Katalysatorlösung (1 mL) wird mittels µL-Spritze zugegeben, sodass sich eine Einwaage von Pd(acac)₂ (11,9 mg, 0,2 mol%) und (**1**) (34,01 mg, 0,4mol%) ergibt. Für Untersuchungen mit flüssigen Säuren wird die benötigte Menge an Säurelösung hinzugeben. Beispielsweise wird für ein H₂SO₄-Verhältnisses von 1:1 0,1 mL (0,4 mol%) mittels µL-Spritze hinzugegeben. Abschließend wird Methanol mittels µL-Spritze hinzugegeben damit ein Gesamtvolumen von 3,94 mL vorliegen und sich ein molares MeOH zu Substrat Verhältnis von 5:1 ergibt. Im zuvor genannten Beispiel werden demnach 2,8 mL zugegeben. Fünf der vorbereiteten Glasgefäße werden in einem 300 mL Autoklaven aufgehangen. Gleichzeitig wird in jedes Gefäß eine separate Leitung geführt, die eine definierte Zudosierung des Substrates (3mL, 19,4 mmol) bei Reaktionstemperatur ermöglicht. Der Autoklav wird verschlossen und dreimal mit CO gespült und dann mit 15 bar CO aufgepresst. Die Reaktionslösungen werden dann auf die benötigte Temperatur von 115 °C aufgeheizt. Nach 20 Minuten bei konstanter Temperatur wird das Substrat mittels HPLC-Pumpe in die Reaktionsgefäße überführt (3 mL, 19,4 mmol). Nach 1 h wird über die Substratleitung jeweils eine Probe gezogen. 0,5 mL dieser Probe werden mit Isooctan als Standard versetzt und Ausbeute und n:iso Verhältnisse mittels GC GCMS-Analyse bestimmt.

| Säure | Säure:(**1**) [mol:mol] | Ausbeute (TMH-ME) [%] |
|---|---|---|
| H₂SO₄ | 1 | 80 |
| | 2,5 | 88 |
| | 4 | 91 |
| | 7 | 89 |
| | 10 | 87 |
| | 13 | 81 |
| | 15 | 70 |
| Al(OTf)₃ | 1 | 84 |
| | 2,5 : 1* | 92 |
| | 4 : 1* | 92 |
| | 7 : 1* | 95 |
| | 10 : 1* | 94 |
| | 13 : 1* | 92 |
| | 15 : 1* | 93 |

| | | |
|---|---|---|
| * erfindungsgemäße Ausführungsbeispiele | | |

## Patentansprüche

1. Verfahren umfassend die Verfahrensschritte:
a) Vorlegen einer ethylenisch ungesättigten Verbindung;
b) Zugabe eines Liganden gemäß Formel (I): 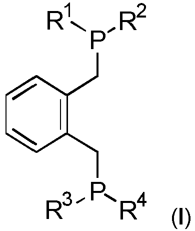 wobei
R¹ und R³ jeweils für einen -(C₃-C₂₀)-Heteroarylrest stehen,
R² und R⁴ jeweils für -(C₁-C₁₂)-Alkyl stehen,
und einer Verbindung, welche Pd umfasst;
c) Vorbehandlung von Aluminiumtriflat durch anlegen von Vakuum;
d) Zugabe des vorbehandelten Aluminiumtriflats aus c),
wobei das Verhältnis Aluminiumtriflat : Ligand im Bereich von 2 mol : 1 mol bis 25 mol : 1 mol liegt;
e) Zugabe eines Alkohols;
f) Zuführen von CO;
g) Erwärmen des Reaktionsgemisches aus a) bis f), wobei die ethylenisch ungesättigte Verbindung zu einem Ester umgesetzt wird.

2. Verfahren nach Anspruch 1,
wobei der Verfahrensschritt c) mindestens zweimal durchgeführt wird, und das angelegte Vakuum durch Fluten mit Inertgas aufgehoben wird.

3. Verfahren nach einem der Ansprüche 1 bis 2,
wobei R¹, R³ jeweils ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

4. Verfahren nach einem der Ansprüche 1 bis 3,
wobei R² und R⁴ für *^{ter}*Bu stehen.

5. Verfahren nach einem der Ansprüche 1 bis 4,
wobei der Ligand in Verfahrensschritt b) die Formel (**1**) aufweist:

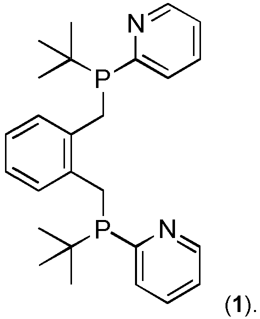

6. Verfahren nach einem der Ansprüche 1 bis 5,
wobei das Verfahren den zusätzlichen Verfahrensschritt c') umfasst:
c') Lösen des vorbehandelten Aluminiumtriflat aus c) in einem Lösungsmittel.

7. Verfahren nach Anspruch 6,
wobei als Lösungsmittel im Verfahrensschritt c') der gleiche Alkohol wie in Verfahrensschritt e) verwendet wird.

8. Verfahren nach einem der Ansprüche 1 bis 7,
wobei die Verbindung in Verfahrensschritt b), welche Pd umfasst, ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

9. Verfahren nach einem der Ansprüche 1 bis 8,
wobei der Alkohol in Verfahrensschritt e) ausgewählt ist aus Methanol, Ethanol, 1-Propanol, 1-Butanol, 1-Pentanol, 1-Hexanol, 2-Propanol, tert-Butanol, 3-Pentanol, Cyclohexanol, Phenol, oder Mischungen davon.

10. Verfahren nach einem der Ansprüche 1 bis 9,
wobei der Alkohol in Verfahrensschritt e) Methanol ist.

11. Verfahren nach einem der Ansprüche 1 bis 10,
wobei das Verhältnis Aluminiumtriflat : Ligand in Verfahrensschritt d) im Bereich von 2,5 mol : 1 mol bis 15 mol : 1 mol liegt.

## Claims

1. Process comprising the process steps of:
a) initially charging an ethylenically unsaturated compound;
b) adding a ligand of formula (I): 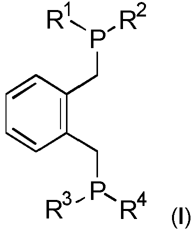 where
R¹ and R³ are each a -(C₃-C₂₀)-heteroaryl radical,
R² and R⁴ are each -(C₁-C₁₂)-alkyl,
and a compound comprising Pd;
c) pretreating aluminium triflate by applying reduced pressure;
d) adding the pretreated aluminium triflate from c), where the ratio of aluminium triflate: ligand is in the range from 2 mol: 1 mol to 25 mol: 1 mol;
e) adding an alcohol;
f) supplying CO;
g) heating the reaction mixture of a) to f), with conversion of the ethylenically unsaturated compound to an ester.

2. Process according to Claim 1,
wherein process step c) is conducted at least twice, and the vacuum applied is broken by flooding with inert gas.

3. Process according to either of Claims 1 and 2,
where R¹, R³ are each selected from furyl, thienyl, pyrrolyl, oxazolyl, isoxazolyl, thiazolyl, isothiazolyl, imidazolyl, pyrazolyl, furazanyl, tetrazolyl, pyridyl, pyridazinyl, pyrimidyl, pyrazinyl, benzofuranyl, indolyl, isoindolyl, benzimidazolyl, quinolyl, isoquinolyl.

4. Process according to any of Claims 1 to 3,
where R² and R⁴ are ^{ter}Bu.

5. Process according to any of Claims 1 to 4,
wherein the ligand in process step b) has the formula (1):

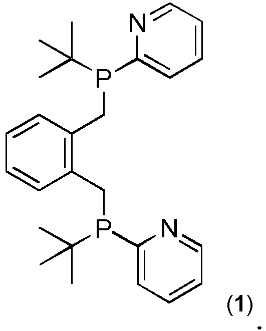

6. Process according to any of Claims 1 to 5,
wherein the process comprises the additional process step c'):
c') dissolving the pretreated aluminium triflate from c) in a solvent.

7. Process according to Claim 6,
wherein the solvent used in process step c') is the same alcohol as used in process step e).

8. Process according to any of Claims 1 to 7,
wherein the compound in process step b) comprising Pd is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), (cinnamyl)palladium dichloride.

9. Process according to any of Claims 1 to 8,
wherein the alcohol in process step e) is selected from methanol, ethanol, 1-propanol, 1-butanol, 1-pentanol, 1-hexanol, 2-propanol, tert-butanol, 3-pentanol, cyclohexanol, phenol, or mixtures thereof.

10. Process according to any of Claims 1 to 9,
wherein the alcohol in process step e) is methanol.

11. Process according to any of Claims 1 to 10,
where the ratio of aluminium triflate: ligand in process step d) is in the range from 2.5 mol: 1 mol to 15 mol: 1 mol.

## Revendications

1. Procédé comprenant les étapes de procédé :
a) disposition préalable d'un composé éthyléniquement insaturé ;
b) ajout d'un ligand selon la formule (I) : 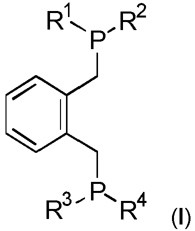 dans laquelle
R¹ et R³ représentent à chaque fois un radical -(C₃-C₂₀)-hétéroalkyle,
R² et R⁴ représentent à chaque fois -(C₁-C₁₂)-alkyle,
et d'un composé, qui comprend du Pd ;
c) prétraitement de triflate d'aluminium par application d'un vide ;
d) ajout du triflate d'aluminium prétraité de c),
le rapport triflate d'aluminium:ligand étant situé dans la plage de 2 moles:1 mole à 25 moles:1 mole ;
e) ajout d'un alcool ;
f) introduction de CO ;
g) chauffage du mélange réactionnel issu de a) à f), le composé éthyléniquement insaturé étant transformé en ester.

2. Procédé selon la revendication 1,
l'étape de procédé c) étant mise en œuvre au moins deux fois et le vide appliqué étant supprimé par injection d'un gaz inerte.

3. Procédé selon l'une des revendications 1 à 2,
R¹, R³ étant choisis à chaque fois parmi furyle, thiényle, pyrrolyle, oxazolyle, isoxazolyle, thiazolyle, isothiazolyle, imidazolyle, pyrazolyle, furazanyle, tétrazolyle, pyridyle, pyridazinyle, pyrimidyle, pyrazinyle, benzofurannyle, indolyle, iso-indolyle, benzimidazolyle, quinoléyle, isoquinoléyle.

4. Procédé selon l'une des revendications 1 à 3,
R² et R⁴ représentant ^{ter}Bu.

5. Procédé selon l'une des revendications 1 à 4,
le ligand dans l'étape de procédé b) présentant la formule (1) :

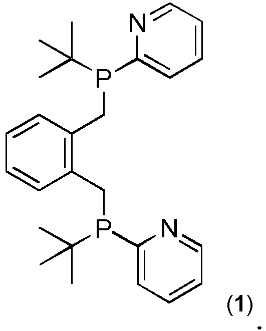

6. Procédé selon l'une des revendications 1 à 5,
le procédé comprenant l'étape de procédé c') supplémentaire :
c') dissolution du triflate d'aluminium prétraité dans un solvant.

7. Procédé selon la revendication 6,
le solvant utilisé dans l'étape de procédé c') étant le même alcool que celui dans l'étape de procédé e).

8. Procédé selon l'une des revendications 1 à 7,
le composé dans l'étape de procédé b), comprenant du Pd, étant choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le (cinnamyl)dichlorure de palladium.

9. Procédé selon l'une des revendications 1 à 8,
l'alcool dans l'étape de procédé e) étant choisi parmi le méthanol, l'éthanol, le 1-propanol, le 1-butanol, le 1-pentanol, le 1-hexanol, le 2-propanol, le tert-butanol, le 3-pentanol, le cyclohexanol, le phénol ou leurs mélanges.

10. Procédé selon l'une des revendications 1 à 9,
l'alcool dans l'étape de procédé e) étant le méthanol.

11. Procédé selon l'une des revendications 1 à 10,
le rapport triflate d'aluminium:ligand dans l'étape de procédé d) étant situé dans la plage de 2,5 moles:1 mole à 15 moles:1 mole.
